# EUROPEAN PATENT SPECIFICATION

(11) **EP 1 745 138 B1**
(45) Date of publication and mention of the grant of the patent: **19.05.2010**
(21) Application number: 05700860.9
(22) Date of filing: 13.01.2005
(51) Int. Cl.: C12P 13/12, C12R 1/15

(54) **METHOD FOR FERMENTATIVE PREPARATION OF METHIONINE BY USE OF RECOMBINANT CORYNEFORM BACTERIA**
VERFAHREN ZUR FERMENTATIVEN HERSTELLUNG VON METHIONIN UNTER VERWENDUNG REKOMBINANTER CORYNEFORMER BAKTERIEN
PROCEDE DE PREPARATION PAR FERMENTATION DE METHIONINE AU MOYEN DE BACTERIES CORYNEFORMES DE RECOMBINAISON

(30) Priority: 27.02.2004 DE 102004009454
(43) Date of publication of application: 24.01.2007
(73) Proprietor: Evonik Degussa GmbH, 45128 Essen (DE)
(72) Inventor: TRÖTSCHEL, Christian, 50767 Köln (DE); KRÄMER, Reinhard, 52428 Jülich (DE); BURKOVSKI, Andreas, 52428 Jülich (DE); BATHE, Brigitte, 33154 Salzkotten (DE)
(86) International application number: PCT/EP2005/000242
(87) International publication number: WO 2005/085463

(56) References cited:
- EP-A- 1 108 790
- WO-A-01/00805
- WO-A-01/00844
- US-A1- 2002 102 664
- KALINOWSKI J ET AL: "THE COMPLETE CORYNEBACTERIUM GLUTAMICUM ATCC 13032 GENONE SEQUENCE AND ITS IMPACT ON THE PRODUCTION OF L-ASPARTATE-DERIVED AMINO ACIDS AND VITAMINS" JOURNAL OF BIOTECHNOLOGY, ELSEVIER SCIENCE PUBLISHERS B.V., AMSTERDAM, NL, vol. 104, no. 1-3, 4 September 2003 (2003-09-04), pages 5-25, XP001184752 ISSN: 0168-1656
- MERLIN CHRISTOPHE ET AL: "The Escherichia coli metD locus encodes an ABC transporter which includes Abc (MetN), YaeE (MetI), and YaeC (MetQ)." JOURNAL OF BACTERIOLOGY. OCT 2002, vol. 184, no. 19, October 2002 (2002-10), pages 5513-5517, XP002325898 ISSN: 0021-9193
- ISNARD A-D ET AL: "The study of methionine uptake in Saccharomyces cerevisiae reveals a new family of amino acid permeases" JOURNAL OF MOLECULAR BIOLOGY, LONDON, GB, vol. 262, no. 4, 1996, pages 473-484, XP002257040 ISSN: 0022-2836
- AYLING P D ET AL: "METHIONINE TRANSPORT IN SALMONELLA-TYPHIMURIUM EVIDENCE FOR AT LEAST 1 LOW AFFINITY TRANSPORT SYSTEM" JOURNAL OF GENERAL MICROBIOLOGY, vol. 114, no. 2, 1979, pages 227-246, XP008046116 ISSN: 0022-1287
- HULLO MARIE-FRANÇOISE ET AL: "The metNPQ operon of Bacillus subtilis encodes an ABC permease transporting methionine sulfoxide, D- and L-methionine." RESEARCH IN MICROBIOLOGY. MAR 2004, vol. 155, no. 2, March 2004 (2004-03), pages 80-86, XP002325899 ISSN: 0923-2508

## Description

The object of invention is a method for the fermentative preparation of L- methionine, by use of recombinant coryneform bacteria, which take up little or no methionine from the surrounding medium. In accordance with the invention this is achieved by attenuated, especially deactivation, of the Met2 methionine uptake system, coded through the genes yaeC, abc and yaeE.

### STATE OF ART

Chemical compounds, by which are meant in particular L-amino acids, vitamins, nucleosides and nucleotides and D-amino acids find use in human medicine, in the pharmaceutical industry, in cosmetics, in the food industry and in animal feeding.

Numerous of these compounds are prepared by fermentation of microorganisms, preferably from strains of coryneform bacteria, prepared especially from the corynebacterium glutamicum.

Because of the great significance, work has proceeded throughout on improvement of manufacturing methods. Process improvements may involve technical features of the fermentation like, for example, agitation and supply of oxygen or the composition of the culture media such as, for example, the sugar concentration during fermentation or production processing by, for example, ion exchange chromatography or the intrinsic output characteristics of the microorganism itself.

For improvement of the performance characteristics of these microorganisms the methods of mutagenesis, selection and choice of mutants are utilized. In this manner strains that are resistant to anti-metabolites, like for example the lysine analog S-(2-aminoethyl)-cysteine or the methionine analogs α-methyl-methionine, ethionine, Norleucine, N-acetylnorleucine, S-trifluoromethylhomocysteine, 2-amino-5-heprenoit acid, seleno-methionine, methioninesulfoxamine, methoxine, 1-aminocyclopentane carboxylic acid, or are auxotrophic for regulatory significant metabolites and produce L-amino acids.

For several years recombinant DNA technology has been employed for strain improvement of amino acid producing strains of corynebacterium glutamicum, in which one amplifies particular amino acid biosynthesis genes and investigates the effect upon the production of L-amino acid.

### OBJECT OF THE INVENTION

The inventors have set themselves the object to provide new foundations for improved methods for the fermentative preparation of L-methionine, with coryneform bacteria.

### DESCRIPTION OF THE INVENTION

L-amino acids or amino acids are mentioned below, meaning therewith one or a plurality of the proteinogenic amino acids including their salts, selected from the group L-aspartic acid, L-asparagine, L-threonine, L-serine, L-glutamic acid, L-glutamine, L-glycine, L-alanine, L-cysteine, L-valine, L-methionine, L-isoleucine, L-leucine, L-tyrosine, L-phenylalanine, L-histidine, L-lysine, L-tryptophan, L-arginine, and L-proline. Particularly preferred is L-methionine.

By proteinogenic amino acids is understood the amino acids which occur in natural proteins, that is in proteins of microorganisms, plants, animals and humans. They serve as structural entities for proteins in which they are linked to each other by peptide bonds.

The L-methionine or methionine mentioned below, refer also to the salts like, for example, methionine hydrochloride or methionine sulfate.

The uptake of methionine from the fermentation medium is very significant for the production of methionine, since a possible re-absorption of the excreted product would lower the production rate. The attenuated genes yaeE, abc and yaeC described in this invention for corynebacterium glutamicum, code the ATP binding protein ABC and the permease YaeE, which together form the MetD2 methionine uptake system, for the periplasmatic binding protein Yaec.

The attenuation, and particularly the deactivation of one or a plurality of the coding genes yaeC, abc and yaeE for the MetD2 methionine uptake system improves the production of L-methionine in the corresponding coryneform bacteria in comparison to the stating organisms without attenuated or deactivation of these genes.

The object of the invention is a method for fermentative preparation of L-methionine by use of recombinant coryneform bacteria, which in particular already produce L-amino acids that take up less methionine than the starting organisms or no methionine from the surrounding medium, and in which one or a plurality of the MetD2 methionine uptake system yaeC, abc and yaeC coding nucleotide sequence(s), is (are) attenuated or in particular deactivated or expressed at a lower level.

A further object of the invention is method for the fermentative preparation of L-methionine in which the following steps are carried out:
(a) Fermentation of the L-methionine acid producing recombinant coryneform bacteria in a medium, wherein less methionine than the starting organisms or no methionine is taken up from the surrounding medium, and in which at least one of the yaeE, abc and yaeC coding genes for the MetD2 methionine uptake system is attenuated, and especially deactivated or is expressed at a lower level,
(b) Enrichment of the L-methionine in the medium or in the cells of the bacteria,
(c) Isolation of the wanted L-methionine, wherein, optionally, components of the fermentation broth and/or the biomass remain in portions (> 0 to 100 %) or in their total amounts in the end product.

The coryneform bacteria produce L-methionine, preferably already before the attenuating or the deactivation of the uptake of methionine from the surrounding medium, which for example is to be accomplished by attenuated or deactivation of one or a plurality of the yaeE, abc and yaeC genes.

It was found that microorganisms, preferably coryneform bacteria, that take up little or no methionine from the surrounding medium, which is achieved by attenuating or deactivation of one or a plurality of the yaeE, abc and yaeC coding genes for the MetD2 methionine uptake system, enhance the production of L-amino acids, especially L-methionine.

The nucleotide sequences of the corynebacterium glutamicum genes mentioned are known to the most recent background art and different patent applications may be cited, along with the data bank of the National Center for Biotechnology Information (NCBI) of the National Library of Medicine (Bethesda, MD, USA).

### yaeC-genes:

- Designation:: Periplasmatic Binding Protein YaeC
- Function:: Part of the MetD2 Methionine Uptake System
- References:: Sequences 7061 and 709 from EP1108790
- Accession No.:: AX127145 and AX120793

### abc-genes:

- Designation:: ATP-Binding Protein ABC
- Function:: Part of the MetD2 Uptake System
- References:: Sequences 7061, 708, 7060 and 707 from EP1108790; Sequence 363 from WO0100805; Sequence 509 from WO010084
- Accession No.:: AX127145; AX120792; AX127144; AX120791; AX066781; AX065383

### yaeE-genes:

- Designation:: Permease YaeE
- Function:: Part of the MetD2 Methionine Uptake System
- References:: Sequences 7060, 7061 and 706 from EP0100805; Sequence 365 from WO0100805
- Accession No.:: AX127144; AX127145; AX120790; AX066783

The sequences coding for the yaeC, abc and yaeE genes described in the given text locations may be used in accordance with the invention. Moreover, allelics of the mentioned gene, which are given through the degenerative nature of the genetic code or by functionally neutral "sense mutations", may be used.

Preferred embodiments can be found in the Claims.

The term "attenuating" or "attenuate" in this context describes the lowering or deactivation of the intracellular activity of one or a plurality of enzyme systems, or proteins in a microorganism, which are coded by the corresponding DNA, in which for example one uses a weak promoter or a gene or allelic, which codes for a corresponding enzyme with a lower activity or the corresponding gene or enzyme or protein inactivates and if necessary combines these features.

By the steps of attenuating, the activity or concentration of the corresponding proteins is in general lowered from 0 to

75 %, 0 to 50 %, 0 to 25 %, 0 to 10 % or 0 to 5 % of the activity or concentration of the wild-type protein, or the activity or concentration of the protein in the starting microorganism.

The information "lesser" relates the same percentages seen in view of the uptake capability of the recombinant microorganisms, in comparison to the starting microorganisms without attenuated or deactivation of the MetD2 methionine uptake system.

The lowering of the protein concentration is detectable by 1- and 2-dimensional common protein separation and subsequent optical identification of the protein concentration in the gel using appropriate evaluation software. A common method for preparation of protein gels for coryneform bacteria and for identification of the proteins is the strategy described in Hermann et al. (Electrophoresis, 22:1712-23 (2001)). The protein concentration can likewise be analyzed by Western-Blot hybridization with a specific antibody for the proving protein (Sambrook et al., Molecular Cloning: A Laboratory Manual. 2nd Ed. Cold Spring Harbor Laboratory Press, NY, (1989)) and subsequent optical interpretation with appropriate software for determination of concentration (Lohaus and Meyer (1998) Biospektrum 5:32-39; Lottspeich, Angewandte Chemie 111: 2630-2647 (1999)). The activity of DNA-binding proteins can be measured using DNA Band Shift Assays (also denoted as gel retardation) as described for example in the textbook "Bioanalytik" (Lottspeich/Zorbas, Spektrum Akademischer Verlag GmbH, Heidelberg, Germany, 1998) and utilized by wilson et al .(J. Bacteriol. 183: 2151-2155 (2001)). The action of DNA binding proteins on the expression of other genes can be proved using different well-described methods of the Reportergen-Assays (Sambrook et al., Molecular Cloning; a Laboratory Manual. 2nd Ed. Cold Spring Harbor Laboratory Press, Cold Spring Harbor, NY, 1989).

The microorganisms which are the object of the present invention can produce amino acids from glucose, saccharose lactose, fructose, maltose, starches, cellulose, or from glycerol and ethanol. Coryneform bacteria, especially the genus corynebacterium can be involved as agent. For the corynebacterium genus, corynebacterium glutamicum is to be mentioned, which is known to experts for its ability to produce L-amino acids.

Suitable strains of the corynebacterium genus, especially the corynebacterium type glutamicum are especially the wild strains.
Corynebacterium glutamicum ATCC13032
Corynebacterium acetoglutamicum ATCC15806
Corynebacterium acetoacidophilum ATCC13870
Corynebacterium melassecola ATCC17965
Corynebacterium thermoaminogenes FERM BP-1539
Brevibacterium flavum ATCC14067
Brevibacterium lactofermentum ATCC13869 and
Brevibacterium divaricatum ATCC14020
Or as for example the L-methionine producing strain
Corynebacterium glutamicum ATCC21608

Strains with the designation "ATCC" may be obtained from the American Type Culture Collection (Manassas, VA, USA).

Strains with the designation "FERM" may be obtained from the National Institute of Advanced Industrial Science and Technology (AIST Tsukuba Central 6, 1-1-1 Higashi, Tsukuba Ibaraki, Japan). The strain of coryne bacterium mentioned thermo amino gene (FERM BP-1539) is described in U.S. Patent 5,250,434.

To obtain a attenuating either the expression of the gene or the catalytic properties of the enzyme proteins can be degraded or deactivated. If necessary both measures can be combined.

The gene expression can be decreased by suitable culture management or by genetic change (mutation) of the signal structures of the gene expression. Signal structures of the gene expression are for example repressor genes, activator genes, operators, promoters, attenuators, ribosome-binding sites, the start codon and terminators. A person skilled in the art can find information for this purpose in patent application WO 96/15246, in the papers by Boyd and Murphy (Journal of Bacteriology 170: 5949-5952 (1988), Vosuil and Chambliss (Nucleic Acids Research 26: 3584-3590 (1998), Patek et al (Microbiology 142: 1297-309 (1996), and Journal of Biotechnology 104: 311-323 (2003)) and in known textbooks of genetics and molecular biology such as, for example, the textbook by Knippers "Moleculare Genetik" (Molecular Genetics), 6th edition, Georg Thieme Verlag, Stuttgart, Germany, 1995), or that by Winnacker ("Gene und Klone" (Genes and Clones), VCH Verlagsgesellschaft, Weinheim, Germany (1990)).

An example of the desired regulation of gene expression is the cloning of genes to be attenuated under control of promoters inducible by addition of closed amounts of IPTG (isopropyl-β-D-thiogalactopyranoside) inducible promoters such as for example the trc promoter or the tac promoter. For this purpose, vectors such as for example the escherichia coli expression vector pXK99E are suitable(WO0226787; filed in accordance with Budapest contract on July 31 2001 as DH5alpha/pXK99E as DSM14440 at the German Association for Microorganisms and Cell Cultures (DSMZ, Braunschweig, Germany) or pVWEx2 (Wendisch, Ph. D thesis Reports of the Jülich Research Center, Jül-3397, ISSN 0994-2952, Jülich, Germany (1997)), which makes an IPTG-dependent expression of the cloned gene in corynebacterium glutamicum possible.

This method was for example included in patent WO0226787 for the regulated expression of the deaD gene by integration of the vector pXK99EdeaD into the genome of corynebacterium glutamicum and by Simic et al. (Applied and Environmental Microbiology 68: 3321-3327 (2002)) to the regulated expression of the glyA gene by integration of the vector pK18mobglyA into corynebacterium glutamicum.

A further method for the specific lowering of the gene expression is antisense technology, wherein short oligodesoxynucleotides or vectors are employed in the synthesis of longer antisense RNA in the target cells.

There the antisense RNA can bind to complementary segments of specific mRNA and lower their stability or block their ability to block the translocator ability. A person skilled in the art can find an example concerning this in Srivastava et al. (Applied Environmental Microbiology, Oct 2000; 66 (10): 4366-4371).

Mutations which lead to a change or lowering of the catalytic properties are known to the state of the art; examples are mentioned in the works of Qiu and Goodman (Journal of Biological Chemistry 272: 8611-8617 (1997)), Sugimoto et al (Bioscience Biotechnology and Biochemistry 61: 1760-1762 (1997)) and mentioned by Möckel (Ph.D thesis, Berichte des Forschungszentrums Jülich (Jülich Research Center Reports), Jül-2906, ISSN09442952, Jülich, Germany (1994). Summary presentations may be found in known textbooks on genetics and microbiology such as for example that in Hagemann ("Allgemeine Genetik"(General Genetics), Gustav Fischer Verlag, Stuttgart (1986).

Transitions, transversions, insertions and deletions come into consideration as mutations. In the dependence of the action of amino acid exchange on enzyme activity "missence mutations" or "nonsense mutations" are spoken about.

Insertions or deletions of at least one base pair in a gene lead to "frame shift mutations", as a consequence of which false amino acids are formed or the translation prematurely terminates. Deletions of a plurality of codons lead typically to a complete breakdown of enzyme activity.

Instructions for the production of such mutations are state of the art and can be found in known textbooks of genetics and microbiology such as for example Knippers ("Molekulare Genetik") (Molecular Genetics), 6th edition, Georg Thieme Verlag, Stuttgart, Germany, 1995), or that by Winnacker ("Gene und Klone" (Genes and Clones), VCH Verlagsgesellschaft, Weinheim, Germany (1990), or that by Hagemann ("Allgemeine Genetik" (General Genetics), Gustav Fischer Verlag, Stuttgart (1986).

A common method of mutating genes of c. glutamicum is the method of "gene disruption" and "gene replacement" described in Schwarzer and Pühler (Bio/Technology 9, 84-87 (1991).

In the method of gene disruption, for example, a central portion of the coding region of the gene of interest is cloned in a plasmid vector, which in one host (typically E. coli) can replicate, but not in c. glutamicum. Vectors that are worth considering are, for example, pSUP301 (Simon et al., Bio/Technology 1, 784-791 (1983)), pK18 mob, pK19mob, pk18mobsacB or pK19mobsacB (Schäfer et al., Gene 145, 69-73 (1994)), pGEM-T (Promega Corporation, Madison, WI, USA, pCR2.1-TOPO (Invitrogen, Groningen, Netherlands); Shuman (1994), Journal of Biological Chemistry 269: 32678-84, U.S. Patent 5,487,993, pCR®Blunt (Invitrogen, Groningen, Netherlands); Bernard et al., Journal of Molecular Biology, 234: 534-541 (1993) or pEM1 (Schrumpf et al., 1991, Journal of Bacteriology 173 4510-4516). The plasmid vector, which contains the central area of the gene coding region, is subsequently converted by conjugation or transformation into the wanted strain of c. glutamicum. The conjugation method is described for example in Schäfer et al. (Journal of Bacteriology 172: 1663-1666 (1990) and Applied and Environmental Microbiology 60: 756-759(1994).

Transformation methods are described for example in Thierbach et al. (Applied Microbiology and Biotechnology 29, 356-362 (1988), Duncan and Shivnan (Bio/Technology 7, 1067-1070 (1989)) and Tauch et al. (FEMS Microbiolgical Letters 123, 343-347 (1994)). After homologous recombination by means of a "cross-over" event, the coding region of the gene in question is interrupted by the vector sequence and one obtains two incomplete allelics each of which lacks the 3' or the 5' ends. This method was used for example by Fitzpatrick et al. (Applied Microbiology and Biotechnology 42, 575-580 (1994) to deactivate the recA-gene of c. glutamicum.

Vectors which contain at least 15 and preferably 25 successive nucleotides of the central portion of the coding region of at least one of the yaeD, abc, or yaeE genes, are likewise a object of the present disclosure.

In the "gene replacement" method, a mutation such as for example a deletion, insertion or base replacement is prepared in the gene of interest in vitro. The prepared allelic on the other hand is cloned in a non-replicative vector for c. glutamicum and this is converted subsequently by transformation or conjugation into the desired host of c. glutamicum. After homologous recombination by means of a first, integration causing "cross-over" event'and an appropriate second, an excision effecting "cross-over" event in the target gene or in the intended sequence one achieves entrapment of the mutation or of the allelic. This method is described in Scharzer and Pühler Bio/Technology 9: 84-87 (1991) and was used for example by Peters-Windisch et al. (Microbiology 144, 915-927 (1998), in order to deactivate the pyc gene of c. glutamicum by a deletion, or by Wehmeier et al. (Microbiology 144: 1853-1862 (1998) for the insertion of a deletion in the rel gene of c. glutamicum.

Kirchner and Tauch (Journal of Biotechnology 104: 287-299 (2003)) provide an overview of different genetic methods for c. glutamicum.

In this manner a deletion, insertion or a base exchange can be built into one or a plurality of the genes selected from the yaeC, abc, and yaeE group.

Furthermore, it can be advantageous for the production of L-amino acids, in addition to decreasing the import of methionine from the surrounding medium, to achieve this, in accordance with the invention, by attenuating one or a plurality of the genes selected from the yaeC, abc and yaE group, one or a plurality of enzymes of the respective biosynthesis routes, of glycolysis, of anaplerotik, of the citric acid cycle, of the pentose phosphate cycle of amino acid export and if necessary regulatory proteins, either to strengthen, or attenuate, especially to deactivate or to decrease the expression.

In this connection, the term "strengthening" or "to strengthen" describes the increase in the intracellular activity or concentration of one or a plurality of enzymes or proteins in a microorganism, which are coded by the corresponding DNA in which, for example, the number of copies of the gene or of the gene is increased, uses a strong promoter or a gene or allelic that for a corresponding enzyme or protein codes with a higher activity, and if necessary combines these measures.

By the measures of strengthening, especially over-expression, the activity or concentration of the corresponding protein increases in general by at least 10 %, 25 %, 50 %, 75 %, 100 %, 150 % 200 %, 300 %, 400 % or 500 %, maximal to 1000 % or 2000 % relative to that of the wild- type protein or the activity or concentration of the protein in the starting microorganism.

The use of endogenous genes is in general preferred.

Under "endogenous genes" or "endogenous nucleotide sequences" is understood the type of genes or nucleotide sequences present in the population.

So, for example, for the preparation of L-methionine, in addition to lowering the import of methionine from the surrounding medium, in accordance with the invention, to achieve by attenuating one or a plurality of the genes, selected from the yaeC, abc, and yaeE group, one or a plurality of the genes chosen from the group of genes or allelics of methionine production is strengthened, especially over-expressed. Under "genes or allelics of methionine production" are altogether, preferably endogenous, open reading frames, genes or allelics to be understood, whose strengthening/over-expression can lead to improvement of methionine production.

For this purpose belong among others the following reading frames, genes or allelics: accBC, accDA, aecD, cstA, cysD, cysE, cysH, cysK, cysN, cysQ, dps, eno, fda, gap, gap2, gdh, gnd, glyA, hom, hom^{FBR}, lysC, lySC^{FBR}, metA, metB, metE, metH, metY, msiK, opcA, oxyR, ppc, ppc^{FBR}, pgk, pknA, pknB, pknD, pknG, ppsA, ptsH, ptsI, ptsM, pyc, pyc P458S, sigC, sigD, sigE, sigH, sigM, tal, thyA, tkt, tpi, zwal, zwf, and zwf A213T, These are summarized and explained in Table 1

**Table 1**

| Genes and Allelics in Methionine Production | | | |
|---|---|---|---|
| Name | Designation of the Coded Enzymes or Proteins | Reference | Accession Number |
| accBC | Acyl-CoA Carboxylase EC 6.3.4.14 (acyl-CoA carboxylase) | Jäger et al. Archives of Microbiology (1996) 166:76-82 EP1108790; WO0100805 | U35023 AX123524 AX066441 |
| accDA | Acetyl-CoA Carboxylase EC 6.4.1.2 (acetyl-CoA carboxylase) | EP1055725 EP1108790 WO0100805 | AX121013 AX066443 |
| aecD | Cystathionin beta-Lyase EC 4.4.1.8 (cystathionine beta-lyase) | Rossol et al., Journal of Bacteriology 174:2968-2977 (1992) | M89931 |
| cstA | Carbon Starvation Protein A (carbon starvation protein A) | EP1108790 WO010080 | AX120811 AX066109 |
| cysD | Sulfat-Adenylyltransferase Untereinheit II EC 2.7.7.4 (sulfate adenylyltransferase small chain) | EP1108790 | AX123177 |
| cysE | Serinacetyltransferase EC 2.3.1.30 (serine acetyltransferase) | EP1108790 WO0100843 | AX122902 AX063961 |
| cysH | 3'-Phosphoadenylsulfat Reduktase EC 1.8.99.4 (3'-phosphoadenosine 5'-phosphosulfate reductase) | EP1108790 WO0100842 | AX123178 AX066001 |
| cysK | Cystein-Synthase EC 4.2.99.8 (cysteine synthase) | EP1108790 WO0100843 | AX122901 AX063963 |
| cysN | Sulfat-Adenylyltransferase Untereinheit I EC 2.7.7.4 (sulfate adenylyltransferase) | EP1108790 | AX123176 AX127152 |
| cysQ | Transportprotein CysQ (transporter cysQ) | EP1108790 WO0100805 | AX12714 5AX066423 |
| dps | DNA-Protection Protein (protection during starvation protein) | EP1108790 | AX127153 |
| eno | Enolase EC 4.2.1.11 (enolase) | EP1108790 WO0100844 EP1090998 Hermann et al., Electrophores is 19:3217-3221 (1998) | AX127146 AX064945 AX136862 |
| fda | Fruktose Bisphosphat Aldolase EC 4.1.2.13 (fructose bisphosphate aldolase) | van der Osten et al., Molecular Microbiology 3:1625-1637 (1989) | X17313 |
| gap | Glyceraldehyd-3-Phosphat Dehydrogenase EC 1.2.1.12 (glyceraldehyde-3-phosphate dehydrogenase) | EP1108790 WO0100844 Eikmanns et al., Journal of Bacteriology 174 : 6076-6086(1992) | AX127148 AX064941 X59403 |
| gap2 | Glyceraldehyd-3-Phosphate Dehydrogenase EC 1.2.1.12 (glyceraldehyde-3-phosphate dehydrogenase 2) | EP1108790 WO0100844 | AX127146 AX064939 |
| gdh | Glutamat Dehydrogenase EC 1.4.1.4 (glutamate dehydrogenase) 11 | EP1108790 WO0100844 Boermann et al., Molecular Microbiology 6:317-326 (1992) | AX127150 AX0638 X59404 X72855 |
| glyA | Glycine/Serin Hydroxymethyltransferase EC 2.1.2.1 (glycine/serine hydroxymethyltransferase) | EP1108790 | AX1271 46 AX1211 94 |
| gnd | 6-Phosphogluconat Dehydrogenase EC 1.1.1.44 (6-phosphogluconate dehydrogenase) | EP1108790 WO0100844 | AX1271 47 AX1216 89 AX0651 25 |
| hom | Homoserin Dehydrogenase EC 1.1.1.3 (homoserine dehydrogenase) | Peoples et al., Molecular Microbiology 2:63-72 (1988) | Y00546 |
| hom^{FBR} | Homoserin Dehydrogenase feedback resistent (fbr) EC 1.1.1.3 (homoserine dehydrogenase fbr) | Reinscheid et al., Journal of Bacteriology 173:3228-30 (1991) | |
| lysC | Aspartatkinase EC 2.7.2.4 (aspartate kinase) | EP1108790 WO0100844 Kalinowski et al., Molecular Microbiology 5:1197-204 (1991) | AX1203 65 AX0637 43 X57226 |
| lysC^{FB} R | Aspartatkinase feedback resistent (fbr) EC 2.7.2.4 (aspartate kinase fbr) | **Siehe Tabelle 2** | |
| metA | Homoserin Acetyltransferase EC 2.3.1.31 (homoserine acetyltransferase) | Park et al., Molecular Cells 8:286- 94 (1998) | AF052652 |
| metB | Cystahionin γ-Lyase EC 4.4.1.1 (cystathionine gamma-synthase) | Hwang et al., Molecular Cells 9:300- 308 (1999) | AF126953 |
| metE | Homocystein-Methyltransferase EC 2.1.1.14 (homocysteine methyltransferase) | EP1108790 | AX1271 46 AX1213 45 |
| metH | Homocystein-Methyltransferase (Vitamin B12 abhängig) EC 2.1.1.14 (homocysteine methyltransferase) | EP1108790 | AX1271 48 AX1217 47 |
| metY | Acetylhomoserin Sulfhydrolase (acetylhomoserine sulfhydrolase) | EP1108790 | AX1208 10 AX1271 45 |
| msiK | Zuckerimporter (multiple sugar import protein) | EP1108790 | AX1208 92 |
| opcA | Glukose-6-Phosphat- Dehydrogenase (subunit of glucose-6- phosphate dehydrogenase) | WO0104325 | AX0762 72 |
| oxyR | Transcriptionsregulator (transcriptional regulator) | EP1108790 | AX1221 98 AX1271 49 |
| ppc^{FBR} | Phosphoenolpyruvat Carboxylase feedback resistent EC 4.1.1.31 (phosphoenolpyruvate carboxylase feedback resistant) | EP0723011 WO0100852 | |
| ppc | Phosphoenolpyruvat Carboxylase EC 4.1.1.31 (phosphoenolpyruvate carboxylase) | EP1108790 O'Reagan et al., Gene 77(2):237- 251(1989) | AX1271 48 AX123554 M25819 |
| pgk | Phosphoglycerat Kinase EC 2.7.2.3 (phosphoglycerate kinase) | EP1108790 WO010084 Eikmanns, Journal of Bacteriology 174:6076-6086 (1992) | AX1218 38 AX1271 48 AX0649 43 X59403 |
| pknA | Protein kinase A (protein kinase A) | EP1108790 | AX1201 31 AX1200 85 |
| pknB | Protein kinase B (protein kinase B) | EP1108790 | AX1201 30 AX1200 85 |
| pknD | Protein kinase D (protein kinase D) | EP1108790 | AX1271 50 AX1224 69 AX1224 68 |
| pknG | Protein kinase G (protein kinase G) | EP1108790 | AX1271 52 AX1231 09 |
| ppsA | Phosphoenolpyruvat-Synthase EC 2.7.9.2 (phosphoenolpyruvate synthase) | EP1108790 | AX1271 44 AX1207 00 AX1224 69 |
| ptsH | Phosphotransferase System Protein H EC 2.7.1.69 (phosphotransferase system component H) | EP1108790 WO0100844 | AX1222 10 AX1271 49 AX0691 54 |
| ptsI | Phosphotransferase System Enzym I EC 2.7.3.9 (phosphotransferase system enzyme I) | EP1108790 | AX1222 06 AX1271 49 |
| ptsM | Glukose spezifisches Phosphotransferase System Enzym II EC 2.7.1.69 (glucose-phosphotransferase- system enzyme II) | Lee et al., FEMS Microbiology Letters 119(1-2):137- 145 (1994) | L18874 |
| pyc | Pyrvat-Carboxylase EC 6.4.1.1 (pyrvate carboxylase) | WO9918228 Peters-Wendisch et al., Microbiology 144:915-927 (1998) | A97276 Y09548 |
| pyc P458S | Pyrvat-Carboxylase EC 6.4.1.1 (pyrvate carboxylase) Amino acid exchange P458S | EP1108790 | |
| sigC | Sigmafaktor C EC 2.7.7.6 (extracytoplasmic function alternative sigma factor C) | EP1108790 | AX120368 AX120085 |
| sigD | RNA Polymerase Sigmafaktor D EC 2.7.7.6 (RNA polymerase sigma factor) | EP1108790 | AX120753 AX127144 |
| sigE | Sigmafaktor E EC 2.7.7.6 (extracytoplasmic function alternative sigma factor E) | EP1108790 | AX127146 AX121325 |
| sigH | Sigmafaktor H EC 2.7.7.6 (sigma factor SigH) | EP1108790 | AX127145 AX120939 |
| sigM | Sigmafaktor M EC 2.7.7.6 (sigma factor SigM) | EP1108790 | AX123500 AX127153 |
| tal | Transaldolase EC 2.2.1.2 (transaldolase) | WO0104325 | AX076672 |
| thyA | Thymidylat Synthase EC 2.1.1.45 (thymidylate synthase) | EP1108790 | AX121026 AX1271 45 |
| tkt | Transketolase EC 2.2.1.1 (transketolase) | Ikeda et al., NCBI | AB023377 |
| tpi | Triose-phosphat Isomerase EC 5.3.1.1 (triose-phosphate isomerase) | Eikmanns, Journal of Bacteriology 174:6076-6086 (1992) X59403 | |
| zwa1 | Zellwachstumsfaktor 1 (growth factor 1) | EP1111062 | AX1337 81 |
| zwf | Glukose-6-phosphat-1- Dehydrogenase EC 1.1.1.49 (glucose-6-phosphate-1- dehydrogenase) | EP1108790 WO010432 | AX127148 AX121827 AX0762 72 |
| zwf A213T | Glukose-6-phosphat-1- Dehydrogenase EC 1.1.1.49 (glucose-6-phosphate-1- dehydrogenase) amino acid exchange? A213T | EP1108790 | |

Moreover, it can be advantageous for L-methionine production, in addition to the lowering of the import of methionine from the surrounding medium, to achieve it by attenuated one or a plurality of the genes selected from the yaeC, abc and yaeE group, at the same time to attenuate one or a plurality of the genes selected from the group of genes or allelics, which are not essential for the growth or production of methionine, and especially to deactivate or to lower the expression.

The following open reading frames belong among others for this purpose: brnQ, ccpA1, ccpA2, citA, citB, citE, ddh, gluA, gluB, gluC, gluD, luxR, luxS, lysR1, lysR2, lysR3, menE, metD, metK, pck, pgi, poxB and zwa2. These are summarized and explained in Table 2.

**Table 2**

| Genes and Allelics, which are not Essential for Methionine Production | | | |
|---|---|---|---|
| Gene Name | Designation of Coded Enzymes or Proteins | Reference | Accession Number |
| brnQ | Carrier-Protein verzweigtkettiger Aminosäuren (branched-chain amino acid transport system carrier protein) | Tauch et al., Archives of Microbiology 169(4):303-12 (1998) W00100805 EP1108790 | M89931 AX066841 AX127150 |
| ccpA1 | Katabolit-Kontroll- Protein (catabolite control protein A1) | W00100844 EP1108790 | AX065267 AX127147 |
| ccpA2 | Katabolit-Kontroll- Protein (catabolite control protein A2) | WO0100844 EP1108790 | AX065267 AX121594 |
| citA | Sensor-Kinase CitA (sensor kinase CitA) | EP1108790 | AX120161 |
| citB | Transkriptionsregulator CitB (transcription regulator CitB) | EP1108790 | AX120163 |
| cite | Citrat-Lyase EC 4.1.3.6 (citrate lyase) | WO010084 EP1108790 | AX065421 AX127146 |
| Ddh | Diaminopimelat- Dehydrogenase EC 1.4.1.16 (diaminopimelate dehydrogenase) | Ishino et al., Nucleic Acids Research 15: 3917 (1987) EP1108790 | S07384 AX127152 |
| gluA | Glutamat-Transport ATP-bindendes Protein (glutamate transport ATP- binding protein) | Kronemeyer et al., Journal of Bacteriology 177(5):1152-8 (1995) | X81191 |
| gluB | Glutamat-bindendes Protein (glutamate binding protein) | Kronemeyer et al., Journal of Bacteriology 177(5):1152-8 (1995) | X81191 |
| gluC | Glutamat-Transport Permease (glutamate transport system permease) | Kronemeyer et al., Journal of Bacteriology 177(5):1152-8 (1995) | X81191 |
| gluD | Glutamat-Transport Permease (glutamate transport system permease) | Kronemeyer et al., Journal of Bacteriology 177(5):1152-8 (1995) | X81191 |
| luxR | Transkriptionsregulator LuxR (transcription regulator LuxR) | W00100842 EP1108790 | AX065953 AX123320 |
| luxS | Histidin-Kinase LuxS (histidine kinase LuxS) | EP1108790 | AX123323 AX127153 |
| lysR1 | Transkriptionsregulator LysR1 (transcription regulator LysR1) | EP1108790 | AX064673 AX127144 |
| lysR2 | Transkriptionsaktivator LysR2 (transcription regulator LysR2) | EP1108790 | AX123312 |
| lysR3 | Transkriptionsregulator LysR3 (transcription regulator LysR3) | WO0100842 EP1108790 | AX065957 AX127150 |
| menE | O-Succinylbenzoesäure- CoA-Ligase EC 6.2.1.26 (O-succinylbenzoate- CoA ligase) | WO0100843 EP1108790 | AX064599 AX064193 AX127144 |
| metD | Transkriptionsregulator MetD (transcription regulator MetD) | EP1108790 | AX123327 AX127153 |
| metK | Methionin-Adenosyl- Transferase EC 2.5.1.6 (S-adenosylmethionine synthetase) | WO0100843 EP1108790 | AX063959 AX127148 |
| Pck | Phosphoenolpyruvat- Carboxykinase (phosphoenolpyruvate carboxykinase) | WO0100844 | AJ269506 AX065053 |
| Pgi | Glucose-6-Phosphat- Isomerase EC 5.3.1.9 (glucose-6- phosphate isomerase) | EP1087015 EP1108790 | AX136015 AX127146 |
| poxB | Pyruvat-Oxidase EC 1.2.3.3 (pyruvate oxidase) | W00100844 EP1096013 | AX064959 AX137665 |
| zwa2 | Zellwachstumsfaktor 2 (growth factor 2) | EP1106693 EP1108790 | AX113822 AX127146 |

Finally, it can be advantageous for the production of amino acids, especially L-methionine, in addition to decreasing the import of methionine from the surrounding medium, for example, to accomplish this by attenuating one or a plurality of the genes, selected from the yaeC, abc and yaeE group, to disable undesirable side reactions (Nakayama: "Breeding of Amino Acid Producing Microorganisms" in: Overproduction of Microbial Products, Krumphanzl, Sikyta, Vanek (eds.) Academic Press, London, UK, 1982).

The microorganisms prepared in accordance with the invention are likewise a object of the invention and can be cultivated continuously or discontinuously in batch processes or in fed batch or repeated fed batch processes for the purpose of production of L-amino acids. A summary of known cultivation methods is described in the textbook by Chmiel (Bioprozesstechnik 1. Einführung in die Bioverfahrenstechnik), (Bioprocess Technology 1. Introduction to Bioprocess Technology) (Gustav Fischer Verlag, Stuttgart (1991) or in Lehrbuch von Storhas, Bioreactoren und Periphere Einrichtungen (Textbook by Storhas, (Bioreactors and Ancillary Equipment) (Viewweg Publishers, Braunschweig/Wiesbaden (1994))

The culture medium to be used must satisfy in a suitable manner the claims of the appropriate strains. Descriptions of culture media of different microorganisms are contained in the handbook "Manual of Methods for General Bacteriology" of the American Society for Bacteriology (Washington D.C., USA, (1981)).

As source of carbon, sugars and carbohydrates such as glucose, saccharose, lactose, fructose, maltose, molasses, starch and cellulose, oils and fats such as soy oil, sunflower oil, groundnut oil and coconut oil , fatty acids such as palmitic acid, stearic acid and linoleic acid, alcohols such as glycerol and ethanol and organic acids such as acetic acid are used. These materials can be used alone or as mixtures.

As sources of nitrogen, organic nitrogen containing compounds such as peptone, yeast extract, meat extract, malt extract, maize water, soybean flour and urea or inorganic compounds such as ammonium sulfate, ammonium chloride, ammonium phosphate, ammonium carbonate and ammonium nitrate can be used.

As source of phosphorus, phosphoric acid, potassium hydrogen phosphate or dipotassium hydrogen phosphate or the corresponding sodium containing salts can be used. The culture medium must furthermore contain metal salts which are needed for growth such as, for example, magnesium sulfate or iron sulfate. Finally, in addition to the materials mentioned above, growth materials such as amino acids and vitamins are added. Suitable precursors may also be added to the culture medium. The additive materials may be added to the culture in the form of single charges or be fed in a suitable way during the process of culturing.

For control of the culture's pH, basic compounds such as sodium hydroxide, potassium hydroxide, ammonia or ammonia water, or acid compounds such as phosphoric acid or sulfuric acid can be employed in a suitable manner. For control of foaming, antifoaming agents such as for example fatty acid polyglycol esters are employed. To maintain plasmid stability, selectively acting materials such as antibiotics may be added to the medium. To maintain aerobic conditions, oxygen or oxygen-containing gas mixtures such as air may be fed into the culture. The temperature of the culture is normally between 20 and 45°C, and preferably between 25 and 40 °C. The culturing process is continued until a maximum amount of the desired product has formed.

This target will normally be achieved within 10 to 160 hours.

With the methods of the invention, the output of the bacteria or of the fermentation process in relation to the product concentration (product per unit volume), the product yield (product formed per utilized carbon source), the product formation (product formed per unit volume an time) or other process parameter and combinations thereof can be improved by at least 0.5 %, at least 1 %, or at least 2 %.

Methods for determination of L-amino acids are known to the art. The analyses can be done as described in Spackmann et al. (Analytical Chemistry, 30, (1958), 1185-1190) by anion exchange chromatography with subsequent Ninhydrin derivatization, or they can be done by reversed phase HPLC as described in Lindroth et al. (Analytical Chemistry (1979) 51: 1167-1174). Those skilled in the art will also find information on this in Ashman et al. (in Tschesche (Hrsg), Modern Methods in Protein Chemistry, 155-172, de Gruyter, Berlin 1985).

The process in accordance with the invention serves for the production of L-methionine by fermentation.

The concentration of L-methionine in the end product can if necessary be approached by addition of L-methionine to the wanted level.

## Claims

1. As method for preparation of L-methionine by fermentation of recombinant coryneform bacteria in which one or a plurality of the genes yaeC, abc and yaeE, coding for the MetD2 methionine absorption system is (are) attenuated, wherein the yaeC gene encodes as periplasmatic binding protein YaeC; the abc gene encodes a ATP-binding protein ABC and the yaeE gene encodes a Permease YaeE.

2. The process for fermentative preparation, of L-methionine according to Claims 1, comprising the following steps:
a) Enrichment of L-methionine in the medium or in the bacteria cells, and
b) Isolation of said L-amino acid, wherein optionally, components of the fermentation broth and/or biomass remain in their tonality or in portions in the end product.

3. The method according to claims 1 or 2, **characterized in that** one lowers the expression of the polynucleotide(s), which codes(code) for components of methionine uptake from the surrounding medium, selected from the yaeC, abc and yaeE genes, coding for the methionine uptake system.

4. The method according to Claims 1 or 2, **characterized in that** one decreases the regulating and/or catalytic properties of the polypeptides (enzyme proteins), for which the yaeC, abc and yaeE polynucleotides code.

5. The method Claims 1 or 2, **characterized in that** for the preparation of L-methionine coryneform bacteria are fermented in which, at the same time one strengthens, especially over-expressed, one or a plurality of the genes selected from the group listed in Table 1:
| Name | Designation of the Coded Enzymes or Proteins |
|---|---|
| accBC | Acyl-CoA Carboxylase |
| | EC 6.3.4.14 |
| | (acyl-CoA carboxylase) |
| accDA | Acetyl-CoA Carboxylase |
| | EC 6.4.1.2 |
| | (acetyl-CoA carboxylase) |
| aecD | Cystathionin beta-Lyase |
| | EC 4.4.1.8 |
| | (cystathionine beta-lyase) |
| cstA | Carbon Starvation Protein A |
| | (carbon starvation protein A) |
| cysD | Sulfat-Adenylyltransferase |
| | Untereinheit II |
| | EC 2.7.7.4 |
| | (sulfate adenylyltransferase small |
| | chain) |
| cysE | Serinacetyltransferase |
| | EC 2.3.1.30 |
| | (serine acetyltransferase) |
| cysH | 3'-Phosphoadenylsulfat Reduktase |
| | EC 1.8.99.4 |
| | (3'-phosphoadenosine 5'- |
| | phosphosulfate reductase) |
| cysK | Cystein-Synthase |
| | EC 4.2.99.8 |
| | (cysteine synthase) |
| cysN | Sulfat-Adenylyltransferase |
| | Untereinheit I |
| | EC 2.7.7.4 |
| | (sulfate adenylyltransferase) |
| cysQ | Transportprotein CysQ |
| | (transporter cysQ) |
| dps | DNA-Protection Protein |
| | (protection during starvation |
| | protein) |
| eno | Enolase |
| | EC 4.2.1.11 |
| | (enolase) |
| fda | Fruktose Bisphosphat Aldolase |
| | EC 4.1.2.13 |
| | (fructose bisphosphate aldolase) |
| gap | Glyceraldehyd-3-Phosphat |
| | Dehydrogenase |
| | EC 1.2.1.12 |
| | (glyceraldehyde-3-phosphate |
| | Dehydrogenase) |
| gap2 | Glyceraldehyd-3-Phosphate |
| | Dehydrogenase |
| | EC 1.2.1.12 |
| | (glyceraldehyde-3-phosphate |
| | dehydrogenase 2) |
| gdh | Glutamat Dehydrogenase |
| | EC 1.4.1.4 |
| | (glutamate dehydrogenase) |
| glyA | Glycine/Serin |
| | Hydroxymethyltransferase |
| | EC 2.1.2.1 |
| | (glycine/serine hydroxymethyltransferase) |
| gnd | 6-Phosphogluconat Dehydrogenase |
| | EC 1.1.1.44 |
| | (6-phosphogluconate dehydrogenase) |
| hom | Homoserin Dehydrogenase |
| | EC 1.1.1.3 |
| | (homoserine dehydrogenase) |
| hom^{FBR} | Homoserin Dehydrogenase feedback |
| | resistent (fbr) |
| | EC 1.1.1.3 |
| | (homoserine dehydrogenase fbr) |
| lysC | Aspartatkinase |
| | EC 2.7.2.4 |
| | (aspartate kinase) |
| lysC^{FBR} | Aspartatkinase feedback resistent |
| | (fbr) |
| | EC 2.7.2.4 |
| | (aspartate kinase fbr) |
| metA | Homoserin Acetyltransferase |
| | EC 2.3.1.31 |
| | (homoserine acetyltransferase) |
| metB | Cystahionin γ-Lyase |
| | EC 4.4.1.1 |
| | (cystathionine gamma-synthase) |
| metE | Homocystein-Methyltransferase |
| | EC 2.1.1.14 |
| | (homocysteine methyltransferase) |
| metH | Homocystein-Methyltransferase |
| | (Vitamin B12 abhängig) |
| | EC 2.1.1.14 |
| | (homocysteine methyltransferase) |
| metY | Acetylhomoserin Sulfhydrolase |
| | (acetylhomoserine sulfhydrolase) |
| msiK | Zuckerimporter |
| | (multiple sugar import protein) |
| opCA | Glukose-6-Phosphat-Dehydrogenase |
| | (subunit of glucose-6-phosphate |
| | dehydrogenase) |
| oxyR | Transcriptionsregulator |
| | (transcriptional regulator) |
| ppc^{FBR} | Phosphoenolpyruvat Carboxylase |
| | feedback resistent |
| | EC 4.1.1.31 |
| | (phosphoenolpyruvate carboxylase |
| | feedback resistant) |
| ppc | Phosphoenolpyruvat Carboxylase |
| | EC 4.1.1.31 |
| | (phosphoenolpyruvate carboxylase) |
| pgk | Phosphoglycerat Kinase |
| | EC 2.7.2.3 |
| | (phosphoglycerate kinase) |
| pknA | Protein kinase A |
| | (protein kinase A) |
| pknB | Protein kinase B |
| | (protein kinase B) |
| pknD | Protein kinase D |
| | (protein kinase D) |
| pknG | Protein kinase G |
| | (protein kinase G) |
| ppsA | Phosphoenolpyruvat-Synthase |
| | EC 2.7.9.2 |
| | (phosphoenolpyruvate synthase) |
| ptsH | Phosphotransferase System. Protein H |
| | EC 2.7.1.69 |
| | (phosphotransferase system component |
| | H) |
| ptsI | Phosphotransferase System. Enzym I |
| | EC 2.7.3.9 |
| | (phosphotransferase system enzyme I) |
| ptsM | Glukose spezifisches |
| | Phosphotransferase System Enzym |
| | II |
| | EC 2.7.1.69 |
| | (glucose-phosphotransferase-system |
| | enzyme II) |
| pyc | Pyrvat-Carboxylase |
| | EC 6.4.1.1 |
| | (pyrvate carboxylase) |
| pyc | Pyrvat-Carboxylase |
| P45 | EC 6.4.1.1 |
| 8S | (pyrvate carboxylase) |
| | amino acid exchange P458S |
| sigC | Sigmafaktor C |
| | EC 2.7.7.6 |
| | (extracytoplasmic function |
| | alternative sigma factor C) |
| sigD | RNA Polymerase Sigmafaktor D |
| | EC 2.7.7.6 |
| | (RNA polymerase sigma factor) |
| sigE | Sigmafaktor E |
| | EC 2.7.7.6 |
| | (extracytoplasmic function |
| | alternative sigma factor E) |
| sigH | Sigmafaktor H |
| | EC 2.7.7.6 |
| | (sigma factor SigH) |
| sigM | Sigmafaktor M |
| | EC 2.7.7.6 |
| | (sigma factor SigM) |
| tal | Transaldolase |
| | EC 2.2.1.2 |
| | (transaldolase) |
| thyA | Thymidylat Synthase |
| | EC 2.1.1.45 |
| | (thymidylate synthase) |
| tkt | Transketolase |
| | EC 2.2.1.1 |
| | (transketolase) |
| tpi | Triose-phosphat Isomerase |
| | EC 5.3.1.1 |
| | (triose-phosphate isomerase) |
| zwa1 | Zellwachstumsfaktor 1 |
| | (growth factor 1) |
| zwf | Glukose-6-phosphat-1-Dehy-drogenase |
| | EC 1.1.1.49 |
| | (glucose-6-phosphate-1- |
| | dehydrogenase) |
| zwf | Glukose-6-phosphat-1-Dehydrogenase |
| A21 | EC 1.1.1.49 |
| 3T | (glucose-6-phosphate-1- |
| | dehydrogenase) |
| | Amino acid exchange A213T |

6. The method according to Claims 1 or 2, **characterized in that** for the preparation of L-methionine, one ferments coryneform microorganisms in which at the same time one attenuates one or a plurality of genes, selected from the group listed in Table 2:
| Gene Name | Designation of the Coded Enzymes or Proteins |
|---|---|
| brnQ | Carrier-Protein |
| | verzweigtkettiger |
| | Aminosäuren |
| | (branched-chain amino acid |
| | transport system carrier |
| | protein) |
| ccpA1 | Katabolit-Kontroll-Protein |
| | (catabolite control protein |
| | A1) |
| ccpA2 | Katabolit-Kontroll-Protein |
| | (catabolite control protein |
| | A2) |
| citA | Sensor-Kinase CitA |
| | (sensor kinase CitA) |
| citB | Transkriptionsregulator CitB |
| | (transcription regulator CitB) |
| citE | Citrat-Lyase |
| | EC 4.1.3.6 |
| | (citrate lyase) |
| ddh | Diaminopimelat-Dehydrogenase |
| | EC 1.4.1.16 |
| | (diaminopimelate |
| | dehydrogenase) |
| gluA | Glutamat-Transport ATP- |
| | bindendes Protein |
| | (glutamate transport ATP- |
| | binding protein) |
| gluB | Glutamat-bindendes Protein |
| | (glutamate binding protein) |
| gluC | Glutamat-Transport Permease |
| | (glutamate transport system |
| | permease) |
| gluD | Glutamat-Transport Permease |
| | (glutamate transport system |
| | permease) |
| luxR | Transkriptionsregulator LuxR |
| | (transcription regulator LuxR) |
| luxS | Histidin-Kinase LuxS |
| | (histidine kinase LuxS) |
| lysR1 | Transkriptionsregulator LysR1 |
| | (transcription regulator |
| | LysR1) |
| lysR2 | Transkriptionsaktivator LysR2 |
| | (transcription regulator |
| | LysR2) |
| lysR3 | Transkriptionsregulator LysR3 |
| | (transcription regulator |
| | LysR3) |
| menE | O-Succinylbenzoesäure-CoA- |
| | Ligase |
| | EC 6.2.1.26 |
| | (O-succinylbenzoate-CoA |
| | ligase) |
| metD | Transkriptionsregulator MetD |
| | (transcription regulator MetD) |
| metK | Methionin-Adenosyl-Transferase |
| | EC 2.5.1.6 |
| | (S-adenosylmethionine |
| | synthetase) |
| pck | Phosphoenolpyruvat- |
| | Carboxykinase |
| | (phosphoenolpyruvate |
| | carboxykinase) |
| pgi | Glucose-6-Phosphat-Isomerase |
| | EC 5.3.1.9 |
| | (glucose-6-phosphate |
| | isomerase) |
| poxB | Pyruvat-Oxidase |
| | EC 1.2.3.3 |
| | (pyruvate oxidase) |
| zwa2 | Zellwachstumsfaktor 2 |
| | (growth factor 2) |

7. The method according to with one or a plurality of Claims 1 through 6, **characterized in that** one employs microorganisms of the corynebacterium glutamicum type.

8. Recombinant Coryneform bacteria, in which one or a. plurality of the genes, selected from the yaeC, abc and yaeE group, which code for the uptake of methionine from the surrounding medium, coding for the MetD2 methionine uptake system, is or are attenuated, compared to the starting organism without attenuated MetD2 methionine uptake system, wherein the yaeC gene encodes a periplasmatic binding protein YaeC, the abC gene encodes a ATP-binding protein ABC and the yaeE gene encodes a Permease YaeE.

## Patentansprüche

1. Verfahren zur Herstellung von L-Methionin mittels Fermentation rekombinanter coryneformer Bakterien, in denen eines oder mehrere der Gene yaeC, abc und yaeE, die für das Methionin-Absorptionssystem MetD2 codieren, abgeschwächt ist bzw. sind, wobei das yaeC-Gen für ein periplasmatisches Bindungsprotein YaeC, das abc-Gen für ein ATP-bindendes Protein ABC und das yaeE-Gen für eine Permease YaeE codiert.

2. Verfahren zur fermentativen Herstellung von L-Methionin nach Anspruch 1, wobei man die folgenden Schritte ausführt:
a) Anreicherung von L-Methionin im Medium oder in den Bakterienzellen und
b) Isolierung der L-Aminosäure, wobei gegebenenfalls Bestandteile der Fermentationsbrühe und/oder Biomasse vollständig oder teilweise im Endprodukt verbleiben.

3. Verfahren nach Anspruch 1 oder 2,
**dadurch gekennzeichnet, dass** man die Expression des Polynukleotids bzw. der Polynukleotide, das bzw. die für Komponenten der Methioninaufnahme aus dem umgebenden Medium, ausgewählt aus den für das Methionin-Aufnahmesystem codierenden Genen yaeC, abc und yaeE, codiert bzw. codieren, erniedrigt.

4. Verfahren nach Anspruch 1 oder 2,
**dadurch gekennzeichnet, dass** man die regulierenden und/oder katalytischen Eigenschaften der Polypeptide (Enzymproteine), für die die yaeC-, abc- und yaeE-Polynukleotide codieren, reduziert.

5. Verfahren nach Anspruch 1 oder 2,
**dadurch gekennzeichnet, dass** zur Herstellung von L-Methionin coryneforme Bakterien fermentiert werden, bei denen man gleichzeitig eines oder mehrere der aus der in Tabelle 1 aufgeführten Gruppe ausgewählten Gene:
| Name | Bezeichnung der codierten Enzyme bzw. Proteine |
|---|---|
| accBC | Acyl-CoA-Carboxylase |
| | EC 6.3.4.14 |
| | (acyl-CoA carboxylase) |
| accDA | Acetyl-CoA-Carboxylase |
| | EC 6.4.1.2 |
| | (acetyl-CoA carboxylase) |
| aecD | Cystathionin beta-Lyase |
| | EC 4.4.1.8 |
| | (cystathionine beta-lyase) |
| cstA | Carbon Starvation-Protein A |
| | (carbon starvation protein A) |
| cysD | Sulfat-Adenylyltransferase |
| | Untereinheit II |
| | EC 2.7.7.4 |
| | (sulfate adenylyltransferase small chain) |
| cysE | Serinacetyltransferase |
| | EC 2.3.1.30 |
| | (serine acetyltransferase) |
| cysH | 3'-Phosphoadenylsulfat-Reduktase |
| | EC 1.8.99.4 |
| | (3'-phosphoadenosine 5'-phosphosulfate |
| | reductase) |
| cysK | Cystein-Synthase |
| | EC 4.2.99.8 |
| | (cysteinsynthase) |
| cysN | Sulfat-Adenylyltransferase |
| | Untereinheit I |
| | EC 2.7.7.4 |
| | (sulfate adenylyltransferase) |
| cysQ | Transportprotein CysQ |
| | (transporter cysQ) |
| dps | DNA-Protection-Protein |
| | (protection during starvation protein) |
| eno | Enolase |
| | EC 4.2.1.11 |
| | (enolase) |
| fda | Fructose-Bisphosphat-Aldolase |
| | EC 4.1.2.13 |
| | (fructose bisphosphate aldolase) |
| gap | Glyceraldehyd-3-Phosphat-Dehydrogenase |
| | EC 1.2.1.12 |
| | (glyceraldehyde-3-phosphate dehydrogenase) |
| gap2 | Glyceraldehyd-3-Phosphat-Dehydrogenase |
| | EC 1.2.1.12 |
| | (glyceraldehyde-3-phosphate dehydrogenase 2) |
| gdh | Glutamat-Dehydrogenase |
| | EC 1.4.1.4 |
| | (glytamate dehydrogenase) |
| glyA | Glycine/Serin-Hydroxymethyltransferase |
| | EC 2.1.2.1 |
| | (glycine/serine hydroxymethyltransferase) |
| gnd | 6-Phosphogluconat-Dehydrogenase |
| | EC 1.1.1.44 |
| | (6-phosphogluconat dehydrogenase) |
| hom | Homoserin-Dehydrogenase |
| | EC 1.1.1.3 |
| | (homoserine dehydrogenase) |
| hom^{FBR} | Homoserin-Dehydrogenase Feedback-resistent |
| | (fbr) |
| | EC 1.1.1.3 |
| | (homoserine dehydrogenase fbr) |
| lysC | Aspartatkinase |
| | EC 2.7.2.4 |
| | (aspartate kinase) |
| lysC^{FBR} | Aspartatkinase Feedback-resistent (fbr) |
| | EC 2.7.2.4 |
| | (aspartate kinase fbr) |
| metA | Homoserin-Acetyltransferase |
| | EC 2.3.1.31 |
| | (homoserine acetyltransferase) |
| metB | Cystathionin-γ-Lyase |
| | EC 4.4.1.1 |
| | (cystathionine gamma-synthase) |
| metE | Homocystein-Methyltransferase |
| | EC 2.1.1.14 |
| | (homocysteine methyltransferase) |
| metH | Homocystein-Methyltransferase |
| | (Vitamin B12-abhängig) |
| | EC 2.1.1.14 |
| | (homocysteine methyltransferase) |
| metY | Acetylhomoserin-Sulfhydrolase |
| | (acetylhomoserine sulfhydrolase) |
| msiK | Zuckerimporter |
| | (multiple sugar import protein) |
| opcA | Glucose-6-phosphat-Dehydrogenase |
| | (subunit of glucose-6-phosphate dehydrogenase) |
| oxyR | Transkriptionsregulator |
| | (transcriptional regulator) |
| ppc^{FBR} | Phosphoenolpyruvat-Carboxylase |
| | Feedback-resistent |
| | EC 4.1.1.31 |
| | (phosphoenolpyruvate carboxylase feedback |
| | resistent) |
| ppc | Phosphoenolpyruvat-Carboxylase |
| | EC 4.1.1.31 |
| | (phosphoenolpyruvate carboxylase) |
| pgk | Phosphoglycerat-Kinase |
| | EC 2.7.2.3 |
| | (phosphoglycerate kinase) |
| pknA | Protein-Kinase A |
| | (protein kinase A) |
| pknB | Protein-Kinase B |
| | (protein kinase B) |
| pknD | Protein-Kinase D |
| | (protein kinase D) |
| pknG | Protein-Kinase G |
| | (protein kinase G) |
| ppsA | Phosphoenolpyruvat-Synthase |
| | EC 2.7.9.2 |
| | (phosphoenolpyruvate synthase) |
| ptsH | Phosphotransferase-System-Protein H |
| | EC 2.7.1.69 |
| | (phosphotransferase system component H) |
| ptsI | Phosphotransferase-System-Enzym I |
| | EC 2.7.3.9 |
| | (phosphotransferase system enzyme I) |
| ptsM | Glucose-spezifisches Phosphotransferase- |
| | System-Enzym II |
| | EC 2.7.1.69 |
| | (glucose-phosphotransferase-system enzyme II) |
| pyc | Pyruvat-Carboxylase |
| | EC 6.4.1.1 |
| | (pyruvate carboxylase) |
| pyc | Pyruvat-Carboxylase |
| P45 | EC 6.4.1.1 |
| 8S | (pyruvate carboxylase) |
| | Aminosäureaustausch |
| sigC | Sigmafaktor C |
| | EC 2.7.7.6 |
| | (extracytoplasmic function alternative sigma |
| | factor C) |
| sigD | RNA-Polymerase-Sigmafaktor D |
| | EC 2.7.7.6 |
| | (RNA polymerase sigma factor) |
| sigE | Sigmafaktor E |
| | EC 2.7.7.6 |
| | (extracytoplasmic function alternative sigma |
| | factor E) |
| sigH | Sigmafaktor H |
| | EC 2.7.7.6 |
| | (sigma factor SigH) |
| sigM | Sigmafaktor M |
| | EC 2.7.7.6 |
| | (sigma factor SigM) |
| tal | Transaldolase |
| | EC 2.2.1.2 |
| | (transaldolase) |
| thyA | Thymidylat-Synthase |
| | EC 2.1.1.45 |
| | (thymidylate synthase) |
| tkt | Transketolase |
| | EC 2.2.1.1 |
| | (transketolase) |
| tpi | Triosephosphat-Isomerase |
| | EC 5.3.1.1 |
| | (triosephosphate isomerase) |
| zwa1 | Zellwachstumsfaktor 1 |
| | (growth factor 1) |
| zwf | Glucose-6-phosphat-1-Dehydrogenase |
| | EC 1.1.1.49 |
| | (glucose 6-phosphate 1-dehydrogenase) |
| zwf | Glucose-6-phosphat-1-Dehydrogenase |
| A21 | EC 1.1.1.49 |
| 3T | (glucose 6-phosphate 1-dehydrogenase) |
| | Aminosäureaustausch A213I |
stärkt, vor allem überexprimiert.

6. Verfahren nach Anspruch 1 oder 2,
**dadurch gekennzeichnet, dass** man zur Herstellung von L-Methionin coryneforme Mikroorganismen fermentiert, bei denen man gleichzeitig ein oder mehrere Gene, ausgewählt aus der in Tabelle 2 aufgeführten Gruppe:
| Genname | Bezeichnung der codierten Enzyme bzw. Proteine |
|---|---|
| brnQ | Carrier-Protein |
| | verzweigtkettiger Aminosäuren |
| | (branched-chain amino acid transport system |
| | carrier protein) |
| ccpA1 | Katabolit-Kontroll-Protein |
| | (catabolite control protein A1) |
| ccpA2 | Katabolit-Kontroll-Protein |
| | (catabolite control protein A2) |
| citA | Sensor-Kinase CitA |
| | (sensor kinase CitA) |
| citB | Transkriptionsregulator CitB |
| | (transcription regulator CitB) |
| citE | Citrat-Lyase |
| | EC 4.1.3.6 |
| | (citrate lyase) |
| ddh | Diaminopimolat-Dehydrogenase |
| | EC 1.4.1.16 |
| | (diaminopimelate dehydrogenase) |
| gluA | ATP-bindendes Protein des Glutamat- |
| | Transports |
| | (glutamate transport ATP-binding protein) |
| gluB | Glutamat-bindendes Protein |
| | (glutamate binding protein) |
| gluC | Glutamat-Transport-Permease |
| | (glutamate transport system permease) |
| gluD | Glutamat-Transport-Permease |
| | (glutamate transport system permease) |
| luxR | Transkriptionsregulator LuxR |
| | (transcription regulator LuxR) |
| luxS | Histidin-Kinase LuxS |
| | (histidine kinase LuxS) |
| lysR1 | Transkriptionsregulator LysR1 |
| | (transcription regulator LysR1) |
| lysR2 | Transkriptionsregulator LysR2 |
| | (transcription regulator LysR2) |
| lysR3 | Transkriptionsregulator LysR3 |
| | (transcription regulator LysR3) |
| menE | O-Succinylbenzoesäure-CoA-Ligase |
| | EC 6.2.1.26 |
| | carrier protein) |
| metD | Transkriptionsregulator MetD |
| | (transcription regulator MetD) |
| metK | Methionin-Adenosyl-Transferase |
| | EC 2.5.1.6 |
| | (S-adenosylmethionine synthetase) |
| pck | Phosphoenolpyruvat-Carboxykinase |
| | (phosphoenolpyruvate carboxykinase) |
| pgi | Glucose-6-phosphat-Isomerase |
| | EC 5.3.1.9 |
| | (glucose 6-phosphate isomerase) |
| poxB | Pyruvat-Oxidase |
| | EC 1.2.3.3 |
| | (pyruvate oxidase) |
| zwa2 | Zellwachstumsfaktor 2 |
| | (growth factor2) |
abschwächt.

7. Verfahren nach einem oder mehreren der Ansprüche 1 bis 6, **dadurch gekennzeichnet, dass** man Mikroorganismen der Art Corynebacterium glutamicum einsetzt.

8. Rekombinante coryneforme Bakterien, in denen eines oder mehrere der Gene, ausgewählt aus der Gruppe yaeC, abc und yaeE, die für die Aufnahme von Methionin aus dem umgebenden Medium codieren, codierend für das Methionin-Aufnahmesystem MetD2, im Vergleich mit dem Ausgangsorganismus ohne abgeschwächtes MetD2-Methionin-Aufnahmesystem abgeschwächt ist bzw. sind, wobei das yaeC-Gen für ein periplasmatisches Bindungsprotein YaeC, das abc-Gen für ein ATP-bindendes Protein ABC und das yaeE-Gen für eine Permease YaeE codiert.

## Revendications

1. Procédé pour la préparation de L-méthionine par fermentation de bactéries corynéformes recombinantes dans lesquelles un ou une pluralité des gènes yaeC, abc et yaeE, codant pour le système d'absorption de méthionine MetD2 est (sont) atténué(s), où le gène yaeC code pour une protéine de liaison périplasmique YaeC, le gène abc code pour une protéine de liaison d'ATP ABC et le gène yaeE code pour une perméase YaeE.

2. Procédé pour la préparation par fermentation de L-méthionine selon la revendication 1, comprenant les étapes suivantes .
a) Enrichissement en L-méthionine dans le milieu ou dans les cellules bactériennes, et
b) Isolement dudit acide aminé L, où, facultativement, les composants du bouillon de fermentation et/ou de la biomasse restent dans leur totalité ou en portions dans le produit final.

3. Procédé selon les revendications 1 ou 2, **caractérisé en ce qu'**on abaisse l'expression du/des polynucléotide(s), qui code(nt) pour des composants d'absorption de méthionine depuis le milieu environnant, choisis parmi les gènes yaeC, abc et yaeE, codant pour le système d'absorption de méthionine.

4. Procédé selon les revendications 1 ou 2, **caractérisé en ce qu'**on diminue les propriétés régulatrices et/ou catalytiques des polypeptides (protéines enzymatiques), pour lesquels les polynucléotides yaeC, abc et yaeE codent.

5. Procédé des revendications 1 ou 2, **caractérisé en ce que** pour la préparation de L-méthionine, des bactéries corynéformes sont fermentées dans lesquelles, simultanément on renforce, en particulier surexprime, un ou une pluralité des gènes choisis dans le groupe répertorié dans le tableau 1 :
| Nom | Désignation des enzymes ou protéines codés |
|---|---|
| accBC | Acyl-CoA Carboxylase |
| | EC 6.3.4.14 |
| | (acyl-CoA carboxylase) |
| accDA | Acetyl-CoA Carboxylase |
| | EC 6.4.1.2 |
| | (acétyl-CoA carboxylase) |
| aecD | Cystathionin beta-Lyase |
| | EC 4.4.1.8 |
| | (cystathionine bêta-lyase) |
| cstA | Carbon Starvation Protein A |
| | (protéine A de privation de carbone) |
| cysD | Sulfat-Adenylyltransferase |
| | Untereinheif-II. EC 2.7.-7.4 |
| | (sulfate adénylyltransférase à petite |
| | chaîne) |
| cysE | Serinacetyltransferase |
| | EC 2.3.1.30 |
| | (sérine acétyltransférase) |
| cysH | 3'-Phosphoadenylsulfat Reduktase |
| | EC 1.8.99.4 |
| | (3'-phosphoadénosine 5'- |
| | phosphosulfate réductase) |
| cysK | Cystein-Synthase |
| | EC 4.2.99.8 |
| | (cystéine synthase) |
| cysN | Sulfat-Adenylyltransferase |
| | Untareinheit I |
| | EC 2.7.7.4 |
| | (sulfate adénylyle transférase) |
| cysQ | Transportprotein CysQ |
| | (transporteur cysQ) |
| dps | Protéine de protection d'ADN |
| | (protection pendant une privation de |
| | protéine) |
| eno | Enolase |
| | EC 4.2.1.11 |
| | (énolase) |
| fda | Fruktose Bisphosphat Aldolase |
| | EC 4.1.2.13 |
| | (fructose bisphosphate aldolase) |
| gap | Glyceraldehyd-3-Phosphat Dehydrogenase |
| | EC 1.2.1.12 |
| | (glycéraldéhyde-3-phosphate |
| | déshydrogénase) |
| gap2 | Glyceraldehyd-3-Phosphate Dehydrogenase |
| | EC 1.2.1.12 |
| | (glycéraldéhyde-3-phosphate |
| | déshydrogénase 2) |
| gdh | Glutamat Dehydrogenase |
| | EC 1.4.1.4 (glutamate déshydrogénase) |
| glyA | Glycine/Serin |
| | Hydroxymethyltransferase |
| | EC 2.1.2.1 |
| | (glycine/sérine hydroxyméthyltransférase) |
| gnd | 6-Phosphogluconat Dehydrogenase |
| | EC 1.1.1.44 |
| | (6-phosphogluconate déshydrogénase) |
| hom | Homoserin Dehydrogenase |
| | EC 1.1.1.3 |
| | (homosérine déshydrogénase) |
| hom^{FBR} | Homoserin Dehydrogenase feedback |
| | resistent (fbr) |
| | EC 1.1.1.3 |
| | (homosérine déshydrogénase fbr) |
| lysC | Aspartatkinase |
| | EC 2.7.2.4 |
| | (aspartate kinase) |
| lysC^{FBR} | Aspartatkinase feedback resistent |
| | (fbr) |
| | EC 2.7.2.4 |
| | (aspartate kinase fbr) |
| metA | Homoserin Acetyltransferase |
| | EC 2.3.1.31 |
| | (homosérine acétyltransférase) |
| metB | Cystathionin γ-Lyase |
| | EC 4.4.1.1 |
| | (cystathionine gamma-synthase) |
| metE | Hcmocystein-Methyltransferase |
| | EC 2.1.1.14 |
| | (homocystéine méthyltransférase) |
| metH | Homocystein-Methyltransferase |
| | (Vitamin B12 abhängig) |
| | EC 2.1.1.14 |
| | (homocystéine méthyltransférase) |
| metY | Acetylhomoserin Sulfhydrolase |
| | (acétylhomosérine sulfhydrolase) |
| msiK | Zuckerimporter |
| | (protéine d'importation de glucide |
| | multiple) |
| opcA | Glukose-6-Phosphat-Dehydrogenase |
| | (sous-unité de glucose-6-phosphate |
| | déshydrogénase) |
| oxyR | Transcriptionsregulator |
| | (régulateur transcriptionnel) |
| ppc^{FBR} | Phosphoenolpyruvat Carboxylase feedback |
| | resistent |
| | EC 4.1.1.31 |
| | (phosphoénolpyruvate carboxylase |
| | résistante à la rétroaction) |
| ppc | Phosphoenolpyruvat Carboxylase |
| | EC 4.1.1.31 |
| | (phosphoénolpyruvate carboxylase) |
| pgk | Phosphoglycerat Kinase |
| | EC 2.7.2.3 |
| | (phosphoglycérate kinase) |
| pknA | Protein kinase A |
| | (protéine kinase A) |
| pknB | Protein kinase B |
| | (protéine kinase B) |
| pknD | Protein kinase D |
| | (protéine kinase D) |
| pknG | Protein kinase G |
| | (protéine kinase G) |
| ppsA | Phosphoenolpyruvat-Synthase |
| | EC 2.7.9.2 |
| | (phosphoénolpyruvate synthase) |
| ptsH | Phosphotransferase System Protein H. |
| | EC 2.7.1.69 |
| | (système phosphotransférase, composant H) |
| ptsI | Phosphotransferase System Enzym I |
| | EC 2.7.3.9 |
| | (enzyme I du système phosphotransférase) |
| ptsM | Glukose spezifisches Phosphotransferase |
| | System Enzym II |
| | EC 2.7.1.69 |
| | (enzyme II du système phosphotransférase) |
| pyc | Pyrvat-Carboxylase |
| | EC 6.4.1.1 |
| | (pyruvate carboxylase) |
| pyc | Pyrvat-Carboxylase |
| P45 | EC 6.4.1.1 |
| 8S | (pyruvate carboxylase) |
| | échange d'acide aminé P458S |
| sigC | Sigmafaktor C |
| | EC 2.7.7.6 |
| | (facteur sigma C alternatif de fonction |
| | extracytoplasmique) |
| sigD | RNA Polymerase Sigmafaktor D |
| | EC 2.7.7.6 |
| | (facteur sigma d'ARN polymérase) |
| sigE | Sigmafaktor E |
| | EC 2.7.7.6 |
| | (facteur sigma E alternatif de fonction |
| | extracytoplasmique) |
| sigH | Sigmafaktor H |
| | EC 2.7.7.6 |
| | (facteur sigma SigH) |
| sigM | Sigmafaktor M |
| | EC 2.7.7.6 |
| | (facteur sigma SigM) |
| tal | Transaldolase |
| | EC 2.2.1.2 |
| | (transaldolase) |
| thyA | Thymidylat Synthase |
| | EC 2.1.1.45 |
| | (thymidylate synthase) |
| tkt | Transketolase |
| | EC 2.2.1.1 |
| | (transcétolase) |
| tpi | Triose-phosphat Isomerase |
| | EC 5.3.1.1 |
| | (triose-phosphate isomérase) |
| zwal | Zellwachstumsfaktor 1 |
| | (facteur de croissance 1) |
| zwf | Glukose-6-phosphat-1-Dehydrogenase |
| | EC 1.1.1.49 |
| | (glucose-6-phosphate-1-déshydrogénase) |
| zwf | Glukose-6-phosphat-1-Dehydrogenase |
| A21 | EC 1.1.1.49 |
| 3T | (glucose-6-phosphate-1-déshydrogénase) |
| | échange d'acide aminé A213T |

6. Procédé selon les revendications 1 ou 2, **caractérisé en ce que** pour la préparation de L-méthionine, on fermente des micro-organismes corynéformes, dans lesquels simultanément on atténue un ou une pluralité de gènes, choisis dans le groupe répertorié dans le tableau 2 :
| Nom | Désignation des enzymes ou protéines codés |
|---|---|
| brnQ | Carrier-Protein verzweigtkettiger |
| | Amino säuren |
| | (protéine porteuse de système de transport |
| | d'acide aminé à chaîne ramifiée) |
| ccpA1 | Katabolit-Kontroll-Protein |
| | (protéine de contrôle de catabolite A1) |
| ccpA2 | Katabolit-Kontroll-Protein |
| | (protéine de contrôle de catabolite A2) |
| CitA | Sensor-Kinase CitA |
| | (capteur kinase CitA) |
| citB | Transkriptionsregulator CitB |
| | (régulateur de transcription CitB) |
| CitE | Citrat-Lyase |
| | EC 4.1.3.6 |
| | (citrate lyase) |
| ddh | Diaminopimelat-Dehydrogenase |
| | EC 1.4.1.16 |
| | (diaminopimélate déshydrogénase) |
| gluA | Glutamat-Transport ATP- |
| | bindendes Protein |
| | (protéine de liaison d'ATP de transport de |
| | glutamate) |
| gluB | Glutamat-bindendes Protein |
| | (protéine de liaison de glutamate) |
| gluC | Glutamat-Transport Permease |
| | (perméase du système de transport de |
| | glutamate) |
| gluD | Glutamat-Transport Permease |
| | (perméase du système de transport de |
| | glutamate) |
| luxR | Transkriptionsregulator LuxR |
| | (régulateur de transcription LuxR) |
| luxS | Histidin-Kinase LuxS |
| | (histidine kinase LuxS) |
| lysR1 | Transkriptionsregulator LysR1 |
| | (régulateur de transcription LysR1) |
| lysR2 | Transkriptionsaktivator LysR2 |
| | (régulateur de transcription LysR2) |
| lysR3 | Transkriptionsregulator LysR3 |
| | (régulateur de transcription LysR3) |
| menE | O-Succinylbenzoesäure-CoA- |
| | Ligase |
| | EC 6.2.1.26 |
| | (O-succinylbenzoate-CoA ligase) |
| metD | Transkriptionsregulator MetD |
| | (régulateur de transcription MetD) |
| metK | Methionin-Adenosyl-Transferase |
| | EC 2.5.1.6 |
| | (S-adénosylméthionine synthétase) |
| pck | Phosphoénolpyruvat-Carboxykinase |
| | (phosphoénolpyruvate carboxykinase) |
| pgi | Glucose-6-Phosphat-Isomerase |
| | EC 5.3.1.9 |
| | (glucose-6-phosphate isomérase) |
| poxB | Pyruvat-Oxidase |
| | EC 1.2.3.3 |
| | (pyruvate oxydase) |
| zwa2 | Zellwachstumsfaktor 2 |
| | (facteur de croissance 2) |

7. Procédé selon une ou une pluralité des revendications 1 à 6, **caractérisé en ce qu'**on utilise des micro-organismes du type Corynebacterium glutamicum.

8. Bactéries corynéformes recombinantes, dans lesquelles un ou une pluralité de gènes choisis dans le groupe yaeC, abc et yaeE, qui codent pour l'absorption de méthionine depuis le milieu environnant, codant pour le système d'absorption de méthionine MetD2, est ou sont atténués, par rapport à l'organisme de départ sans système d'absorption de méthionine MetD2 atténué, où le gène yaeC code pour une protéine de liaison périplasmique YaeC, le gène abc code pour une protéine de liaison d'ATP ABC et le gène yaeE code pour une perméase YaeE.
